# EUROPEAN PATENT APPLICATION

(11) **EP 3 835 416 A1**
(43) Date of publication of application: **16.06.2021**
(21) Application number: 19848755.5
(22) Date of filing: 12.08.2019
(51) Int. Cl.: C12N 5/0783, G01N 33/569, A01N 1/02, A61K 35/17, A61P 35/00

(54) **METHOD FOR PREPARING AND CRYOPRESERVING CANCER ANTIGEN-SPECIFIC CD8+ T CELLS**

(30) Priority: 10.08.2018 US 201862717258 P
(71) Applicant: Eutilex Co., Ltd., Seoul 08594 (KR)
(72) Inventor: KWON, Byoung Se, Seoul 08594 (KR); KIM, Young Ho, Seoul 08594 (KR); KIM, Mun Ki, Seoul 08594 (KR); KIM, Kwang Hee, Seoul 08594 (KR); KANG, You Hyun, Seoul 08594 (KR); LEE, Sae Rom, Seoul 08594 (KR)
(74) Representative: D Young & Co LLP
(86) International application number: PCT/KR2019/010242
(87) International publication number: WO 2020/032782

(57) **Abstract**

The present invention relates to a method of preparing cancer antigen-specific CD8+ T cells comprising (a) activating the CD8+ T cells from among PBMCs comprising cancer antigen-specific CD8+ T cells, and then expressing a co-stimulatory factor of these CD8+ T cells; (b) selecting the CD8+ T cells expressing a co-stimulatory factor among the cells activated in step (a); (c) increasing the number of CD8+ T cells selected in step (b); (d) freezing the CD8+ T cells obtained in step (c); and (e) thawing the CD8+ T cells that were frozen in step (d).

## Description

### Technical field

The present invention relates to the art of isolating, proliferating and cryopreserving antigen-specific CD8+ T cells.

### Background art

Because CD8+ T cells have a comparatively simple function compared to other cells such as dendritic cells, CD4+ T and NK cells, unexpected side effects in anti-cancer immunotherapy are less likely to occur. In general, MHC class I/peptide multimers are used to isolate antigen-specific CD8+ T cells, but this method has the drawback that because the rate of cell death due to apoptosis after cell isolation is high, in order to produce a sufficient quantity of antigen-specific CD8+ T cells, it is necessary to culture them over a long period. Therefore, a surrogate marker capable of isolating antigen-specific CD8+ T cells by replacing the MHC multimers that stimulate T cell receptor (TCR) is required; for this purpose, the protein 4-1BB (CD137) is used.

4-1BB is an inducible co-stimulatory molecule and is expressed in activated T cells; in particular, stimulation via 4-1BB is known not only to enhance the activity of CD8+ T cells, but also to act to inhibit activation-induced cell death (AICD) by increasing the expression of anti-apoptotic molecules such as Bcl-2, Bcl-XL, and Bfl-1 and the like.

The present inventors have previously disclosed methods of isolating and proliferating antigen-specific CD8+ T cells using the expression of 4-1BB by activated CD8+ T cells, or using an anti-4-1BB antibody or pentameric COMP-4-1BBL protein (Republic of Korea Registered Patents Nos. 100882445, 101103603, 101503341). The background for the present specification is the T cell or method of manufacturing the same disclosed in the above patents.

This patent document discloses a technique for isolating and mass-culturing the antigen-specific CD8+ T cells for foreign antigens in the form of virus antigens (EBV/LMP2A, CMV/pp65) or autologous antigens (WT-1, hTERT, NY-ESO-1, and the like). However, in existing techniques for isolating antigen-specific CD8+ T cells using a plate coated with anti-4-1BB antibody coated plate, the purity of the isolated cells varies greatly with the skill of the operator, and the process of culturing the cells is also very complex. Moreover, because the production process takes 30 days or more, ongoing work is required to shorten as much as possible the time needed to produce cell therapeutics for current clinical applications.

In addition, in preparing CD8+ T cells, although it is necessary for reasons such as the patient's treatment schedule and repeated treatment to cryopreserve the cells and thawing them when needed, but to date, there has been the problem that the T cells' survival rate, proliferative capacity, and cancer antigen-specific activity are reduced when the T cells are frozen and thawed.

Accordingly, for more effective patient treatment, it is necessary to isolate antigen-specific CD8+ T cells more easily and with high purity in a short time to shorten the whole culture process to proliferate them quickly, and to develop a method of cryopreserving T cells that minimizes degradation in T cell performance.

### Detailed Description of the Invention

### Problem to be solved

The present invention seeks to provide a method for producing cancer antigen-specific CD8+ T cells that enables maintaining a high level of cell viability and cancer antigen-specific activity even after the produced CD8+ T cells are cryopreserved.

The present invention seeks to provide a CD8+ T cell-containing pharmaceutical composition prepared by a method comprising a cryopreservation step.

The present invention seeks to provide a method wherein frozen antigen-specific CD8+ T cells are thawed and mass culturing is again performed, so that CD8+ T cells may be obtained with greater antigen specificity while also maintaining high levels of cell viability and cancer antigen-specific activity.

### Means of solving the problem

1. A method of preparing cancer antigen-specific CD8+ T cells, comprising (a) activating the CD8+ T cells from among PBMCs comprising cancer antigen-specific CD8+ T cells, and then expressing a co-stimulatory factor of these CD8+ T cells; (b) selecting the CD8+ T cells expressing a co-stimulatory factor among the cells activated in step (a); (c) increasing the number of CD8+ T cells selected in step (b); and (d) freezing the CD8+ T cells obtained in step (c).
2. A method of preparing cancer antigen-specific CD8+ T cells according to Item 1, further comprising a step (e), thawing the CD8+ T cells that were frozen in step (d).
3. A method of preparing cancer antigen-specific CD8+ T cells according to Item 1, wherein the cancer antigen is at least one selected from the group made up of hTERT, NY-ESO1, MAGE-A3, WT1 and EBV.
4. A method of preparing cancer antigen-specific CD8+ T cells according to Item 1, wherein step (b) is performed using a closed-system flow cytometer.
5. A method of preparing cancer antigen-specific CD8+ T cells according to Item 1, wherein in step (d), freezing is performed when the number of CD8+ T cells expressing a co-stimulatory factor is greater than or equal to a predetermined number of cells per mL.
6. A method of preparing cancer antigen-specific CD8+ T cells according to Item 1, wherein in step (d), freezing is performed when the number of CD8+ T cells is at least a predetermined number of cells per mL, and the proportion of CD8+ T cells expressing the co-stimulatory factor is at least a predetermined ratio.
7. A method of preparing cancer antigen-specific CD8+ T cells according to Item 1, wherein step (d) comprises a step of detecting the number of CD8+ T cells and the proportion of CD8+ T cells expressing a co-stimulatory factor.
8. A method of preparing cancer antigen-specific CD8+ T cells according to Item 2, further comprising a step (f) of increasing the number of CD8+ T cells obtained in step (e).
9. A method of preparing cancer antigen-specific CD8+ T cells according to Item 1, wherein the co-stimulatory factor is one or more selected from the group made up of CD28, CTLA-4, ICOS, PD1, BTLA, HVEM, CD27, OX40, 4-1BB, CD30, and SLAM.
10. A method of preparing a cancer treatment composition, comprising (a) activating the CD8+ T cells from among PBMCs comprising cancer antigen-specific CD8+ T cells, and then expressing a co-stimulatory factor of these CD8+ T cells; (b) selecting the CD8+ T cells expressing a co-stimulatory factor among the cells activated in step (a); (c) increasing the number of CD8+ T cells selected in step (b); and (d) freezing the CD8+ T cells obtained in step (c).
11. A method of preparing a cancer treatment composition according to Item 10, further comprising a step (e), thawing the CD8+ T cells that were frozen in step (d).
12. A cancer treatment composition comprising cancer antigen-specific CD8+ T cells prepared by steps comprising (a) activating the CD8+ T cells from among PBMCs comprising cancer antigen-specific CD8+ T cells, and then expressing a co-stimulatory factor of these CD8+ T cells; (b) selecting the CD8+ T cells expressing a co-stimulatory factor among the cells activated in step (a); (c) increasing the number of CD8+ T cells selected in step (b); and (d) freezing the CD8+ T cells obtained in step (c).
13. A cancer treatment composition according to Item 12, further comprising a step (e), thawing the CD8+ T cells that were frozen in step (d).
14. A cancer treatment method comprising a step of administering a pharmaceutically effective amount of a cancer treatment composition comprising cancer antigen-specific CD8+ T cells prepared by steps comprising (a) activating the CD8+ T cells from among PBMCs comprising cancer antigen-specific CD8+ T cells, and then expressing a co-stimulatory factor of these CD8+ T cells; (b) selecting the CD8+ T cells expressing a co-stimulatory factor among the cells activated in step (a); (c) increasing the number of CD8+ T cells selected in step (b); and (d) freezing the CD8+ T cells obtained in step (c).
15. A cancer treatment method according to Item 14, further comprising a step (e), thawing the CD8+ T cells that were frozen in step (d).

### Effect of the invention

The CD8+ T cell production method of the present invention makes it possible to cryopreserve CD8+ T cells without degrading their performance.

The CD8+ T cell production method of the present invention makes it possible to maintain a high level of cell viability and cancer antigen-specific activity even the CD8+ T cells have been cryopreserved.

The CD8+ T cell production method of the present invention makes it possible to selectively isolate and mass-culture cancer antigen-specific CD8+ T cells with high purity.

The CD8+ T cell production method of the present invention makes it possible to rapidly produce cancer antigen-specific CD8+ T cells.

The CD8+ T cell production method of the present invention may be applied in a GMP process.

The CD8+ T cell-containing pharmaceutical composition of the present invention makes it possible to maintain high levels of cell viability and cancer antigen-specific activity even after cryopreservation.

The CD8+ T cell-containing pharmaceutical composition of the present invention is able to comprise cancer antigen-specific CD8+ T cells at a high degree of purity.

### Brief description of the drawings

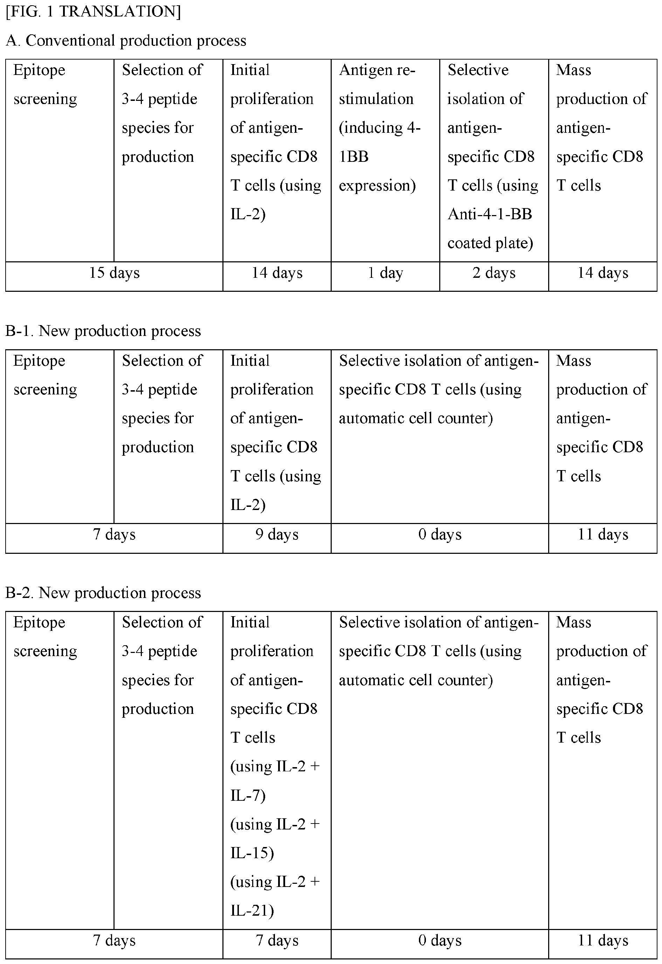

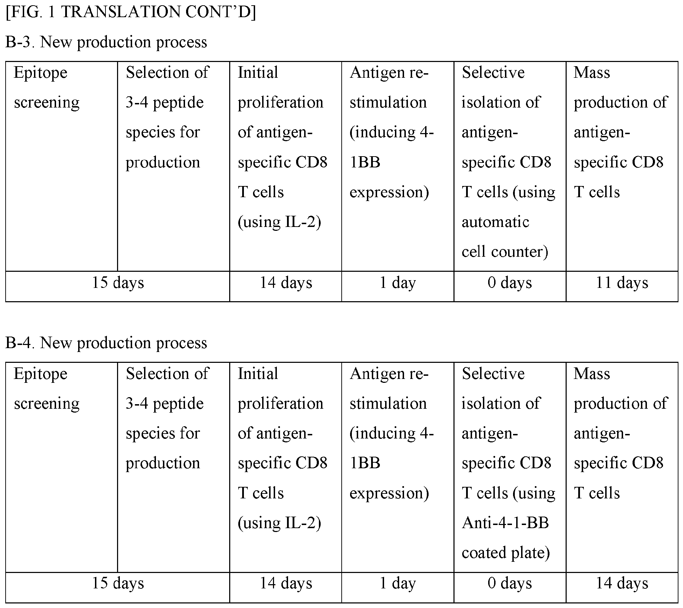

FIG. 1 schematically depicts a process for producing cancer antigen-specific CD8+ T cells.

### Best mode of implementing the invention

The present invention relates to a method of cryopreserving T cells and to T cells produced by such a method.

The present invention relates to a method for producing cancer antigen-specific CD8+ T cells that enables maintaining a high level of cell viability and cancer antigen-specific activity even after the produced CD8+ T cells are cryopreserved, and to a T cell therapeutic produced by such a method.

The pharmaceutical composition of the present invention comprises cytotoxic T cells specific for cancer antigens (for example, EBV, CMV, hTERT, NY-ESO-1, WT1, MAGE-A3, Neo-Antigen, etc.); the time taken from primary proliferation to freezing after completion of the final T cell mass culture may be 31 days; an improved production process may take 29 days, 26 days, 20 days or 15 days.

The method of preparing cancer antigen-specific CD8+ T cells according to the present invention comprises (a) activating the CD8+ T cells from among PBMCs comprising cancer antigen-specific CD8+ T cells, and then expressing a co-stimulatory factor of these CD8+ T cells; (b) selecting the CD8+ T cells expressing a co-stimulatory factor among the cells activated in step (a); (c) increasing the number of CD8+ T cells selected in step (b); (d) freezing the CD8+ T cells obtained in step (c); and (e) thawing the CD8+ T cells that were frozen in step (d).

CD8+ T cells are activated in the following way:
In order to activate CD8+ T cells, PBMCs isolated from blood and respective peptides derived from cancer antigens are cultured together, and are periodically treated with at least one cytokine from the group made up of IL-2, IL-7, IL-15 and IL-21.

Co-stimulatory factors of CD8+ T cells are proteins belonging to the CD28 family, for example CD28, CTLA-4, ICOS, PD1, and BTLA; proteins belonging to the TNFR family are, for example, HVEM, CD27, 0X40, 4-1BB, GITR and CD30; and proteins belonging to the SLAM family are for example SLAM, 2B4, CD2, CD48, CD84, CD229, CRACC, NTB-A; and proteins belonging to the TIM family are for example TIM-1 and TIM-3; but they are not limited thereto (K. C. Beier et al., European Respiratory Journal 2007 29: 804-812). In one embodiment of the present invention, the co-stimulatory factor is 4-1BB.

Activated CD8+ cells expressing a co-stimulatory factor may alternatively be prepared by the preparation method below.

One embodiment comprises a) a step of first culturing a PBMC (peripheral blood mononuclear cell) isolated from the blood of a cancer patient with a respective peptide derived from a cancer antigen, and screening activated T cells to screen for cancer antigen CD8+ T cell epitopes; and b) a step of culturing the PBMCs isolated from the blood of cancer patients in a medium comprising at least one cytokine selected from the group made up of IL-2, IL-7, IL-15 and IL-21 and the screened epitope, thereby inducing 4-1BB expression in the PBMC.

In the method according to the present application, the step of CD8+ T cell epitope sorting or screening is intended to increase the avidity for cancer cells or alternatively to increase the concentration of cancer antigen-specific CD8+ T cells that are present in blood at a low concentration of 0.1% or below, and is the first step that enables selectively proliferating and isolating cancer antigen-specific CD8+ T cells.

In this step, epitopes from various cancer antigens (for example EBV, CMV, hTERT, NY-ESO-1, WT1, MAGE-A3, etc.) that CD8+ cells are able to recognize may be used. However, this varies depending on the individual patient's condition. In other words, even if the same type of cancer antigen in cancer antigens such as for example EBV, CMV, hTERT, NY-ESO-1, WT1, MAGE-A3, etc., the part that may act as epitope depends on each patient's HLA-A type and condition. Therefore, even within the same cancer antigen, it is important to select and use the cancer antigen CD8+ T cell epitopes present in the blood of individual cancer patients through epitope screening, because the peptide portions that may act as antigen epitopes are different for each cancer patient. Generally, for use in preparing T cell therapeutics, 3-4 types of peptides are selected that act as epitopes.

The method according to the present application selects epitopes that are optimal for selecting and proliferating CD8+ T cells in individuals, from a variety of cancer antigens; various cancer antigens that achieve this objective may be used, including both autologous (self) and non-self antigens.

For example, autologous cancer antigens derived from the patient's own genes include hTERT (GenBank: BAC11010.1), WT1 (GenBank: AAO61088.1), NY-ESO1 (GenBank: CAA05908.1), MAGE-A3 (NCBI Reference Sequence : NP_005353.1) and cancer-specific mutant antigens such as neoantigens, mutated P53, RAS, and the like may include tumor suppressor or trigger genes, and foreign cancer antigens such as carcinogenic virus antigens such as CMV, EBV, HPV and the like.

However, the characteristics of the invention of the present application, which selects an optimal epitope for each individual from various cancer antigens specific for each type of cancer and uses these to produce T cells, are not limited hereto. Known autologous cancer antigens include for example WT1, hTERT, NY-ESO1, Mesothelin, MAGE's, and the like. For example, hTERT, a self-type cancer antigen, is an enzyme that synthesizes telomeric DNA at the termini of chromosomes, which cancer cells excessively activate to evade telomere-dependent cell death, and which is known as a target antigen for various solid cancers including lung, stomach and pancreatic cancer (Kim NW, et al. Science. 1994; 266: 2011-2015); WT1 is a gene associated with Wilms tumor and encodes a zinc finger transcription factor, a protein that is involved in cell proliferation, differentiation, apoptosis, and organ development, and is known as a target antigen for cerebrospinal cancer, lung cancer and the like (Call KM, et al., Cell. 1990. 60: 509-520; Nakahara Y, et al., Brain Tumor Pathol. 2004. 21: 113-6). In addition, the aforementioned NY-ESO1 is one of the proteins belonging to the cancer testis antigens (CTA), and is known to be expressed chiefly in germ cells, sarcoma, and various cancer cells including breast cancer (Gnjatic S, et al., Adv Cancer Res. 2006; 95:1-30). MAGE-A3 is a protein belonging to the melanoma-associated antigen family; the function it performs in healthy cells is unknown, but it is known to overexpress in various cancer cells including lung cancer, sarcoma and melanoma, and is being evaluated as a suitable target antigen for cancer immunotherapy (Decoster L, et al., Ann Oncol. 2012 Jun; 23 (6): 1387-93). Accordingly, in the method of the present application, cancer antigens known to be associated with particular cancers may be used for producing individual cancer antigen CD8+ T cells.

In addition, viral cancer antigens are known, including for example EBV, HPV, MC polyoma virus, HBV, HCV, and CMV; Epstein-Barr virus antigen is known as a target antigen for Hodgkin's lymphoma, nasopharyngeal carcinoma, stomach cancer, Burkitt's lymphoma, and NK/T lymphoma, and human papilloma virus antigens are known as target antigens of uterine cancer and head and neck cancer, while MC polyoma virus is known as the target antigen of Merkel cell cancer. Hepatitis B and C viruses are also known as target cells for liver cancer.

As tissue-specific cancer antigens, tyrosinase, GP100, and MNRT-1 are known as melanoma target antigens, and PSMA, PAP, and PSA are known as prostate cancer target antigens.

Two, three, four, five or more peptides are used that are derived from cancer antigens. In particular, considering that 3-4 types of peptides acting as epitopes are generally used for the preparation of T cell therapeutics, the peptides derived from cancer antigens used number at least 4 or more, 5 or more, 6 or more, 7 or more, 8 or more, 9 or more, or more, but are not limited hereto, and may be def to the determination of the skilled person in view of the features of the method of the present application and the art of the relevant field.

The epitope screening step that the method of the present application comprises differs from the conventional epitope screening process in that during screening, epitopes may be selected by a method of screening activated T cells (for example, 4-1BB + CD8+ T cells). For example, after antigen stimulation, activated T cells are selected, and antigen epitopes that are able to induce such cells may be selected by detecting the presence and amount of activated T cells (for example, 4-1BB + CD8+ T cells) that are present. The activated T cells (for example, 4-1BB + CD8+ T cells) may be screened using an automatic cell sorter. The automatic cell sorter may be one that is able to be used in a GMP process. For example, it may be a closed-system automatic cell sorter.

In the next step, 4-1BB expression is induced in the PBMCs isolated from cancer patient blood by culturing these PBMCs in a medium comprising a combination of the selected epitopes and cytokines.

In one embodiment according to the present application, the cytokine may be any one selected from the group made up of IL-2, IL-7, IL-15 and IL-21. In another embodiment, the cytokine may be a combination of at least two selected from the group made up of IL-2, IL-7, IL-15, and IL-21. For example, the cytokine may be a combination of IL-2 and IL-7, IL-2 and IL-15, IL-2 and IL-21, IL-7 and IL-15, IL-7 and IL-21, or IL-15 and IL-21.

The proliferation of conventional cancer antigen-specific CD8+ T cells is carried out over 14 days of culture, and on day 14 of culture, additional reactivation for 24 hours is required in order to induce 4-1BB expression in antigen-specific CD8+ T cells.

In this embodiment, step b) of the present application may be performed over a period of 7, 8, 9, 10, 11, 12, 13 or 14 days.

In view of the purpose of the present application, the peripheral blood mononuclear cells (PBMCs) used in the first and second steps of the method according to the present application are from the same patient. PBMCs may be isolated from whole blood using methods known in the art.

Next, the CD8+ T cells expressing the co-stimulatory factor among the activated CD8+ T cells are isolated after staining with an anti-co-stimulatory factor antibody and anti-CD8 antibody. Isolation may be performed using, for example, an automatic cell sorter.

The isolation of CD8+ T cells expressing antigen-specific co-stimulatory factors using an automatic cell sorter may improve several problems such as purity and difficulty of work caused by the conventional process using a plate coated with co-stimulatory factor. After staining with a co-stimulatory factor for an anti-CD8 antibody and a marker that is expressed only on the surface of CD8+ T cells stimulated by the antigen coupled to a fluorescent labeling agent, the antigen-specific CD8+ T cells that have both kinds of fluorescence may efficiently be isolated.

As described above, the preparation method in whichCD8+ T cells expressing a co-stimulatory factor are selected using an automatic cell sorter may be used in the preparation of CD8+ T cells that are specific for cancer antigens such as EBV, hTERT, NY-ESO-1, WT1, and MAGE-A3. As described above, the pharmaceutical composition prepared by the method of selecting CD8+ T cells expressing a co-stimulatory factor using an automatic cell sorter may comprise at least 90% of cancer antigen-specific cytotoxic T cells (for example, 90%, 91%, 92%, 93%, 94%, 95%, 96%, 97%, 98%, or 99%). More preferably, it may comprise at least 95% (for example, 95%, 96%, 97%, 98%, or 99%).

A pharmaceutical composition (for example, a composition comprising cytotoxic T cells that are specific for EBV, CMV, hTERT, NY-ESO-1, WT1, or MAGE-A3) prepared by a preparation method in which CD8+ T cells expressing a co-stimulatory factor are selected using an automatic cell sorter as described above has high purity of the cancer antigen-specific cytotoxic T cells, and thus has superior clinical efficacy (for example, anti-cancer efficacy and the like) as a T cell therapeutic.

The automatic cell sorter used in the above selection step may be one that can be used in a GMP process. For example, it may be a closed-system automatic cell sorter.

The number of screened CD8+ T cells may be increased through culturing, and methods for culturing CD8+ T cells that are known in the art may be used: For example, in one embodiment of the present invention, T cells are plated onto a 6-well plate RPMI medium (inactivated FBS, 1-glutamine, penicillin, streptomycin, 2-mercaptoethanol, 1% ITS: insulin, transferrin and sodium selenite) coated with anti-CD3 and anti-CD28 antibodies and incubated for a certain period, and after incubation upon adding IL-7, and IL-2, the cells were recovered and re-incubated on a fresh medium comprising IL-7 and IL-2. However, the above method is one illustrative example and the invention is not limited thereto.

In another embodiment of the present invention, the number of CD8+ T cells may be increased through culturing: a) a step of first culturing a PBMC (peripheral blood mononuclear cell) isolated from the blood of a cancer patient with a respective peptide derived from a cancer antigen, and screening activated T cells to screen for cancer antigen CD8+ T cell epitopes; b) a step of culturing the PBMCs isolated from the blood of cancer patients in a medium comprising at least one cytokine selected from the group made up of IL-2, IL-7, IL-15 and IL-21 and the screened epitope, thereby inducing 4-1BB expression in the PBMC; and c) a step of staining the cells in which 4-1BB expression was induced with anti-4-1BB antibody and anti-CD8 antibody and then separating them.

Herein below, the method of the present invention for screening antigen epitopes for the production of antigen-specific CD8+ T cells, and isolating and proliferating antigen-specific CD8+ T cells, will be described step by step.

In the method according to the present application, the step of epitope sorting or screening is intended to increase the avidity for cancer cells or alternatively to increase the concentration of cancer antigen-specific CD8+ T cells that are present in blood at a low concentration of 0.1% or below, and is the first step that enables selectively proliferating and isolating cancer antigen-specific CD8+ T cells.

In this step, epitopes may be used that are derived from various cancer antigens (for example EBV, CMV, hTERT, NY-ESO-1, WT1, MAGE-A3, etc.) that may be recognized by CD8+ T cells. However, this will vary depending on the individual patient's condition. In other words, even if the same type of cancer antigen in cancer antigens such as for example EBV, CMV, hTERT, NY-ESO-1, WT1, MAGE-A3, etc., the part that may act as epitope depends on each patient's HLA-A type and condition. Therefore, even within the same cancer antigen, it is important to select and use the cancer antigen CD8+ T cell epitopes present in the blood of individual cancer patients through epitope screening, because the peptide portions that may act as antigen epitopes are different for each cancer patient. Generally, for use in preparing T cell therapeutics, 3-4 types of peptides are selected that act as epitopes.

The method according to the present application comprises selecting epitopes that are optimal for selecting and proliferating CD8+ T cells in individuals, from a variety of cancer antigens; various cancer antigens that achieve this objective may be used, including both autologous (self) and non-self antigens.

For example, autologous cancer antigens derived from the patient's own genes include hTERT (GenBank: BAC11010.1), WT1 (GenBank: AAO61088.1), NY-ESO1 (GenBank: CAA05908.1), MAGE-A3 (NCBI Reference Sequence : NP_005353.1) and cancer-specific mutant antigens such as neoantigens, mutated P53, RAS, and the like may include tumor suppressor or trigger genes, and foreign cancer antigens such as carcinogenic virus antigens such as CMV, EBV, HPV and the like.

However, the characteristics of the invention of the present application, which selects an optimal epitope for each individual from various cancer antigens specific for each type of cancer and uses these to produce T cells, are not limited hereto. Known autologous cancer antigens include for example WT1, hTERT, NY-ESO1, Mesothelin, MAGE's, and the like. For example, hTERT, a self-type cancer antigen, is an enzyme that synthesizes telomeric DNA at the termini of chromosomes, which cancer cells excessively activate to evade telomere-dependent cell death, and which is known as a target antigen for various solid cancers including lung, stomach and pancreatic cancer (Kim NW, et al. Science. 1994; 266: 2011-2015); WT1 is a gene associated with Wilms tumor and encodes a zinc finger transcription factor, a protein that is involved in cell proliferation, differentiation, apoptosis, and organ development, and is known as a target antigen for cerebrospinal cancer, lung cancer and the like (Call KM, et al., Cell. 1990. 60: 509-520; Nakahara Y, et al., Brain Tumor Pathol. 2004. 21: 113-6). In addition, the aforementioned NY-ESO1 is one of the proteins belonging to the cancer testis antigens (CTA), and is known to be expressed chiefly in germ cells, sarcoma, and various cancer cells including breast cancer (Gnjatic S, et al., Adv Cancer Res. 2006; 95:1-30). MAGE-A3 is a protein belonging to the melanoma-associated antigen family; the function it performs in healthy cells is unknown, but it is known to overexpress in various cancer cells including lung cancer, sarcoma and melanoma, and is being evaluated as a suitable target antigen for cancer immunotherapy (Decoster L, et al., Ann Oncol. 2012 Jun; 23 (6): 1387-93). Accordingly, in the method of the present application, cancer antigens known to be associated with particular cancers may be used for producing individual cancer antigen CD8+ T cells.

In addition, viral cancer antigens are known, including for example EBV, HPV, MC polyoma virus, HBV, HCV, and CMV; Epstein-Barr virus antigen is known as a target antigen for Hodgkin's lymphoma, nasopharyngeal carcinoma, stomach cancer, Burkitt's lymphoma, and NK/T lymphoma, and human papilloma virus antigens are known as target antigens of uterine cancer and head and neck cancer, while MC polyoma virus is known as the target antigen of Merkel cell cancer. Hepatitis B and C viruses are also known as target cells for liver cancer.

As tissue-specific cancer antigens, tyrosinase, GP100, and MNRT-1 are known as melanoma target antigens, and PSMA, PAP, and PSA are known as prostate cancer target antigens.

Two, three, four, five or more peptides are used that are derived from cancer antigens. In particular, considering that 3-4 types of peptides acting as epitopes are generally used for the preparation of T cell therapeutics, the peptides derived from cancer antigens used number at least 4 or more, 5 or more, 6 or more, 7 or more, 8 or more, 9 or more, or more, but are not limited hereto, and may be def to the determination of the skilled person in view of the features of the method of the present application and the art of the relevant field.

The epitope screening step that the method of the present application comprises differs from the conventional epitope screening process in that during screening, epitopes may be selected by a method of screening activated T cells (for example, 4-1BB + CD8+ T cells). For example, after antigen stimulation, activated T cells are selected, and antigen epitopes that are able to induce such cells may be selected by detecting the presence and amount of activated T cells (for example, 4-1BB + CD8+ T cells) that are present. The activated T cells (for example, 4-1BB + CD8+ T cells) may be screened using an automatic cell sorter. The automatic cell sorter may be one that is able to be used in a GMP process. For example, it may be a closed-system automatic cell sorter.

In the next step, 4-1BB expression is induced in the PBMCs isolated from cancer patient blood by culturing these PBMCs in a medium comprising a combination of the selected epitopes and cytokines.

Because cancer antigen-specific CD8+ T cells are present in the blood at a low concentration of 0.1% or less, it is possible to proliferate CD8+ T cells specific to peptides derived from cancer antigens and induce 4-1BB expression by adding one or more cytokines and 3-4 peptides selected from epitope screening to PBMCs isolated from blood and then incubating for 7 to 9 days (FIG. 1.B-1, B-2). As a result, the total incubation time may be reduced to 29 days or less, 26 days or less, 20 days or less, 18 days or less, and in particular 15 days.

In one embodiment according to the present application, the cytokine may be any one selected from the group made up of IL-2, IL-7, IL-15 and IL-21. In another embodiment, the cytokine may be a combination of at least two selected from the group made up of IL-2, IL-7, IL-15, and IL-21. For example, the cytokine may be a combination of IL-2 and IL-7, IL-2 and IL-15, IL-2 and IL-21, IL-7 and IL-15, IL-7 and IL-21, or IL-15 and IL-21.

The proliferation of conventional cancer antigen-specific CD8+ T cells is carried out over 14 days of culture, and on day 14 of culture, additional reactivation for 24 hours is required in order to induce 4-1BB expression in antigen-specific CD8+ T cells.

In this embodiment, step b) of the present application may be performed over a period of 7, 8, 9, 10, 11, 12, 13 or 14 days.

In view of the purpose of the present application, the peripheral blood mononuclear cells (PBMCs) used in the first and second steps of the method according to the present application are from the same patient. PBMCs may be isolated from whole blood using methods known in the art.

In a third step, antigen-specific cells induced with 4-1BB expression are isolated after staining with anti-4-1BB antibody and anti-CD8 antibody. Isolation may be performed using, for example, an automatic cell sorter. The isolation of antigen-specific 4-1BB+ CD8+ T cells using an automatic cell sorter may improve several problems caused by the conventional process using a plate coated with 4-1BB antibody, such as purity and difficulty of work. With the 4-1BB protein, after staining with an anti-CD8 antibody and a 4-1BB antibody a marker that is expressed only on the surface of CD8+ T cells stimulated by the antigen and is coupled to a fluorescent labeling agent, the antigen-specific CD8+ T cells that have both kinds of fluorescence may be isolated efficiently.

As described above, the preparation method in which CD8+ T cells expressing 4-1BB+ are selected using an automatic cell sorter may be used in the preparation of CD8+ T cells that are specific for cancer antigens such as EBV, hTERT, NY-ESO-1, WT1, and MAGE-A3. As described above, the pharmaceutical composition prepared by the method of selecting CD8+ T cells expressing 4-1BB+ using an automatic cell sorter may comprise at least 90% of cancer antigen-specific cytotoxic T cells (for example, 90%, 91%, 92%, 93%, 94%, 95%, 96%, 97%, 98%, or 99%).

A pharmaceutical composition (for example, a composition comprising cytotoxic T cells that are specific for BV, CMV, hTERT, NY-ESO-1, WT1, or MAGE-A3) prepared by a preparation method in which CD8+ T cells expressing 4-1BB+ are selected using an automatic cell sorter as described above has high purity of the cancer antigen-specific cytotoxic T cells, and thus has superior clinical efficacy (for example, anti-cancer efficacy and the like) as a T cell therapeutic.

The automatic cell sorter used in the above selection step may be one that can be used in a GMP process. For example, it may be a closed-system automatic cell sorter.

The method according to the present application further comprises a step of mass propagating these isolated 4-1BB+ CD8+ T cells.

The mass production step may comprise a step of culturing the isolated cancer antigen-specific CD8+ T cells and irradiated allogeneic PBMCs in a medium comprising IL-2, anti-CD3 antibody and autologous plasma. In one embodiment, 4-1BB+ CD8+ T cells (5 × 10⁵ - 3 × 10⁶ cells), irradiated allogeneic PBMCs (1 × 10⁸ - 3 × 10⁸ cells) isolated in a 1L culture vessel (culture bag or G-Rex device), 1000 U/mL IL-2, and 30 ng anti-CD3 mAb are mixed and added to medium at regular intervals over 7 to 11 days, and then the cells are mass-cultured so that they proliferate to a level greater than 1 × 10⁹ cells/L at which they may be administered to a cancer patient. In one embodiment of the present invention, proliferation is performed so that cells are comprised at approximately 1 × 10⁶ cells/mL or more, approximately 2 × 10⁶ cells/mL or more, approximately 3 × 10⁶ cells/mL or more, approximately 4 × 10⁶ cells/mL or more, approximately 5 × 10⁶ cells/mL or more, approximately 6 × 10⁶ cells/mL or more, approximately 7 × 10⁶ cells/mL or more, approximately 8 × 10⁶ cells/mL or more, approximately 9 × 10⁶ cells/mL or more, approximately 1 × 10⁷ cells/mL or more, approximately 2 × 10⁷ cells/mL or more, approximately 3 × 10⁷ cells/mL or more, approximately 4 × 10⁷ cells/mL or more, approximately 5 × 10⁷ cells/mL or more, approximately 6 × 10⁷ cells/mL or more, approximately 7 × 10⁷ cells/mL or more, approximately 8 × 10⁷ cells/mL or more, approximately 9 × 10⁷ cells/mL or more, approximately 1 × 10⁸ cells/mL or more, approximately 2 × 10⁸ cells/mL or more, approximately 3 × 10⁸ cells/mL or more, approximately 4 × 10⁸ cells/mL or more, approximately 5 × 10⁸ cells/mL or more, approximately 6 × 10⁸ cells/mL or more, approximately 7 × 10⁸ cells/mL or more, approximately 8 × 10⁸ cells/mL or more, or approximately 9 × 10⁸ cells/mL or more.

The method according to the present application comprises a step of freezing proliferated (expanded) cancer antigen-specific CD8+ T cells.

The freezing step may be carried out by adding a cryopreservative (for example, DMSO) when the number of CD8+ T cells that have been preselected from among the cells proliferated in the preceding step by the expression of a co-stimulatory molecule reaches a predetermined number of cells per mL. This predetermined number of cells may be 1 × 10⁶ cell/mL, 2 × 10⁶ cell/mL, 3 × 10⁶ cell/mL, 4 × 10⁶ cell/mL, 5 × 10⁶ cell/mL, 6 × 10⁶ cell/mL, 7 × 10⁶ cell/mL, 8 × 10⁶ cell/mL, 9 × 10⁶ cell/mL, 1 × 10⁷ cell/mL, 2 × 10⁷ cell/mL, 3 × 10⁷ cell/mL, 4 × 10⁷ cell/mL, 5 × 10⁷ cell/mL, 6 × 10⁷ cell/mL, 7 × 10⁷ cell/mL, 8 × 10⁷ cell/mL, or 9 × 10⁷ cell/mL. However, person of ordinary skill in the art may adjust the freezing time as needed. The DMSO is, for example, added at 10%, 7.5%, 5%, or 2.5%. CD8+ T cells expressing a co-stimulatory molecule may be, for example, a cell in which a co-stimulatory molecule is expressed in the antigen stimulation step, and may also be for example a cell in which a co-stimulatory molecule is expressed in the proliferation step.

In one embodiment of the present invention, the freezing step may be performed when the number of CD8+ T cells is at least a predetermined number of cells per mL, while the proportion of CD8+ T cells selected for co-stimulatory factor expression is at least a predetermined proportion.

This predetermined number of cells may be 1 × 10⁶ cell/mL, 2 × 10⁶ cell/mL, 3 × 10⁶ cell/mL, 4 × 10⁶ cell/mL, 5 × 10⁶ cell/mL, 6 × 10⁶ cell/mL, 7 × 10⁶ cell/mL, 8 × 10⁶ cell/mL, 9 x10⁶ cell/mL, 1 × 10⁷ cell/mL, 2 × 10⁷ cell/mL, 3 × 10⁷ cell/mL, 4 × 10⁷ cell/mL, 5 × 10⁷ cell/mL, 6 × 10⁷ cell/mL, 7 × 10⁷ cell/mL, 8 × 10⁷ cell/mL, or 9 × 10⁷ cell/mL. However, a person of ordinary skill in the art may adjust the timing of freezing as needed. The percentage of CD8+ T cells expressing a co-stimulatory factor may be, for example, 1% to 10%, 10% to 20%, 20% to 30%, 30% to 40%, 40% to 50%, 50% to 60%, 60% to 70%, 70% to 80%, 80% to 90%, or 90% to 100%. To this end, in one embodiment of the present invention, the freezing step according to the present invention may comprise a step of detecting the number of CD8+ T cells and the proportion of CD8+ T cells expressing the co-stimulatory factor. Here, all numerical values in the range are considered to be disclosed in the present specification.

The method according to the present application comprises a step of thawing the frozen cancer antigen-specific CD8+ T cells.

The method according to the present application may further comprise a step of proliferating the thawed cancer antigen-specific CD8+ T cells. For example, the post-thawing proliferation step may be performed in the same or similar manner as the pre-freezing proliferation step. In the post-thawing proliferation step, cells may be mass-cultured at a level of > 1 × 10⁹ cell/mL to proliferate to a level that may be administered to cancer patients.

In one embodiment of the present invention, the cancer antigen may be autologous cancer antigen, viral cancer antigen, or tissue-specific cancer antigen.

For example, autologous cancer antigens derived from the patient's own genes include hTERT (GenBank: BAC11010.1), WT1 (GenBank: AAO61088.1), NY-ESO1 (GenBank: CAA05908.1), MAGE-A3 (NCBI Reference Sequence : NP_005353.1) and cancer-specific mutant antigens such as neoantigens, mutated P53, RAS, and the like may include tumor suppressor or trigger genes, and foreign cancer antigens such as carcinogenic virus antigens such as CMV, EBV, HPV and the like.

However, the characteristics of the invention of the present application, which selects an optimal epitope for each individual from various cancer antigens specific for each type of cancer and uses these to produce T cells, are not limited hereto. Known autologous cancer antigens include for example WT1, hTERT, NY-ESO1, Mesothelin, MAGE's, and the like. For example, hTERT, a self-type cancer antigen, is an enzyme that synthesizes telomeric DNA at the termini of chromosomes, which cancer cells excessively active to evade telomere-dependent cell death, and which is known as a target antigen for various solid cancers including lung, stomach and pancreatic cancer (Kim NW, et al. Science. 1994; 266: 2011-2015); WT1 is a gene associated with Wilms tumor and encodes a zinc finger transcription factor, a protein that is involved in cell proliferation, differentiation, apoptosis, and organ development, and is known as a target antigen for cerebrospinal cancer, lung cancer and the like (Call KM, et al., Cell. 1990. 60: 509-520; Nakahara Y, et al., Brain Tumor Pathol. 2004. 21: 113-6). In addition, the aforementioned NY-ESO1 is one of the proteins belonging to the cancer testis antigens (CTA), and is known to be expressed chiefly in germ cells, sarcoma, and various cancer cells including breast cancer (Gnjatic S, et al., Adv Cancer Res. 2006; 95:1-30). MAGE-A3 is a protein belonging to the melanoma-associated antigen family; the function it performs in healthy cells is unknown, but it is known to overexpress in various cancer cells including lung cancer, sarcoma and melanoma, and is being evaluated as a suitable target antigen for cancer immunotherapy (Decoster L, et al., Ann. Oncol. 2012 Jun; 23 (6): 1387-93). Accordingly, in the method of the present application, cancer antigens known to be associated with particular cancers may be used for producing individual cancer antigen CD8+ T cells.

In addition, viral cancer antigens are known, including for example EBV, HPV, MC polyoma virus, HBV, HCV, and CMV; Epstein-Barr virus antigen is known as a target antigen for Hodgkin's lymphoma, nasopharyngeal carcinoma, stomach cancer, Burkitt's lymphoma, and NK/T lymphoma, and human papilloma virus antigens are known as target antigens of uterine cancer and head and neck cancer, while MC polyoma virus is known as the target antigen of Merkel cell cancer. Hepatitis B and C viruses are also known as target cells of liver cancer.

As tissue-specific cancer antigens, tyrosinase, GP100, and MNRT-1 are known as melanoma target antigens, and PSMA, PAP, and PSA are known as prostate cancer target antigens.

The composition of the present invention comprises a pharmaceutically effective amount of T cells. The effective amount may be readily determined by persons of ordinary skill in the art based on the disclosure in this specification. In general, a pharmaceutically effective amount is determined by 1st administering a low concentration of an active ingredient, and then gradually increasing the concentration until a desired effect is achieved in the subject without any side effects (for example, the symptoms associated with cancer are reduced or eliminated). Methods of determining appropriate dosages or intervals of administration for the administration of the compositions according to the present invention are described, for example, in Goodman and Gilman's The Pharmacological Basis of Therapeutics, Goodman et al., eds., 11th Edition, McGraw-Hill 2005, and Remington: The Science and Practice of Pharmacy, 20th and 21st Editions, Gennaro and University of the Sciences in Philadelphia, Eds., Lippencott Williams & Wilkins (2003 and 2005).

The method of administration of the composition according to the present invention may be determined based on various factors such as the subject's type of cancer, age, weight, sex, medical condition, severity of the disease, route of administration, and other medications to be administered separately. Accordingly, although the method of administration varies widely, it may be determined according to a commonly used method.

The amount of the composition according to the present invention to be administered to a subject may be determined by numerous factors such as the method of administration, subject's state of health, weight, and medical advice; all of these factors are within the scope of knowledge of a person of ordinary skill in the art.

The pharmaceutical composition for preventing or treating cancer comprising the cancer antigen-specific cytotoxic T cell according to the present invention may comprise approximately 1 × 10⁶ cells/mL or more, approximately 2 × 10⁶ cells/mL or more, approximately 3 × 10⁶ cells/mL or more, approximately 4 × 10⁶ cells/mL or more, approximately 5 × 10⁶ cells/mL or more, approximately 6 × 10⁶ cells/mL or more, approximately 7 × 10⁶ cells/mL or more, approximately 8 × 10⁶ cells/mL or more, approximately 9 × 10⁶ cells/mL or more, approximately 1 × 10⁷ cells/mL or more, approximately 2 × 10⁷ cells/mL or more, approximately 3 × 10⁷ cells/mL or more, approximately 4 × 10⁷ cells/mL or more, approximately 5 × 10⁷ cells/mL or more, approximately 6 × 10⁷ cells/mL or more, approximately 7 × 10⁷ cells/mL or more, approximately 8 × 10⁷ cells/mL or more, approximately 9 × 10⁷ cells/mL or more, approximately 1 × 10⁸ cells/mL or more, approximately 2 × 10⁸ cells/mL or more, approximately 3 × 10⁸ cells/mL or more, approximately 4 × 10⁸ cells/mL or more, approximately 5 × 10⁸ cells/mL or more, approximately 6 × 10⁸ cells/mL or more, approximately 7 × 10⁸ cells/mL or more, approximately 8 × 10⁸ cells/mL or more, or approximately 9 × 10⁸ cells/mL or more, but a person of ordinary skill in the art may adjust the concentration of cytotoxic T cells within the range in which the same effects may be obtained.

The cancer antigen is at least one selected from the group made up of OY-TES-1, hTERT, NY-ESO1, MAGE-A3, WT1, PSMA, TARP, mesothelin, tyrosinase, GP100, MNRT-1, PAP, PSA, CMV, HCV, HBV, MC polyoma virus, HPV and EBV. In one embodiment of the present invention, the cancer antigen may be selected from the group made up of hTERT, NY-ESO1, MAGE-A3, WT1 and EBV.

The "composition" disclosed in this invention refers to a combination of the cytotoxic T cells according to the present invention as the active ingredient, and inactive ingredients such as natural or artificial carriers, labels or detectors, an active ingredients such as adjuvants, diluents, coupling agents, stabilizers, buffers, salts, lipophilic solvents, and preservatives, and comprises a pharmaceutically acceptable carrier. The carrier may also comprise pharmaceutical excipients and additional proteins, peptides, amino acids, lipids, and carbohydrates (for example, monosaccharides; disaccharides; trisaccharides; tetrasaccharides; oligosaccharides; alditol, aldonic acid, sugar-derived polysaccharides such as esterified sugar, or a sugar polymer or the like), alone or in combination, at 1 to 99.99 wt% or vol%. Protein excipients include, for example, human serum albumin, recombinant human albumin, gelatin, casein, and the like, but are not limited thereto. Representative amino acid components that may play a buffer role include, for example, alanine, arginine, glycine, betaine, histidine, glutamic acid, aspartic acid, cysteine, lysine, leucine, isoleucine, valine, methionine, phenylalanine, aspartame, and the like, but are not limited thereto. Carbohydrate excipients also include, for example, monosaccharides such as fructose, maltose, galactose, glucose, D-mannose, sorbose; disaccharides such as lactose, sucrose, trehalose, cellobiose; polysaccharides such as raffinose, maltodextrin, dextran, and starch; and alditols such as mannitol, xylitol, maltitol, lactitol, sorbitol, and myoinositol; but are not limited thereto.

A skilled person will be able to formulate the pharmaceutical composition of the present invention by methods known in the art. For example, as required, it may be used parenterally in the form of an injection of a sterile solution or suspension with water or another pharmaceutically acceptable liquid. For example, it may be appropriately combined with pharmaceutically acceptable carriers or media, in particular sterile water or saline solution, vegetable oil, emulsifier, suspension agent, surfactant, stabilizer, excipient, vehicle, preservative, binder and the like; it may be formulated by mixing in a unit-dosage form required by generally accepted pharmaceutical practice. The active ingredient amount used in the formulation is such that a suitable dosage in the indicated range may be obtained.

In addition, sterile compositions for injection may be formulated according to conventional formulation practice using excipient liquids, such as distilled water for injection. As the aqueous solution for injection may be used, for example, combinations of physiological saline; isotonic solutions containing glucose or other auxiliary agents, for example D-sorbitol, D-mannose, D-mannitol, sodium chloride, and suitable dissolution aids, for example alcohols, in particular ethanol, and polyalcohols, for example propylene glycol, polyethylene glycol; and nonionic surfactants such as polysorbate 80 (TM), HCO-50. Oily liquids include for example sesame oil and soybean oil, and may be used in combination with benzyl benzoate and benzyl alcohol as a dissolution aid.

Injection formulations may for example be administered by intravenous injection, intraarterial injection, selective intraarterial injection, intramuscular injection, intraperitoneal injection, subcutaneous injection, intraventricular injection, intracranial injection, intramedullary injection, and the like; preferably, however, they are administered by intravenous injection.

It may also be combined with buffers, for example phosphate buffer solutions or sodium acetate buffer solutions; analgesics, for example procaine hydrochloride; stabilizers, for example benzyl alcohol, phenols and antioxidants. The prepared injection solution is usually charged into a suitable ampoule.

Suspensions and emulsions may contain as carriers, for example, natural gums, agar, sodium alginate, pectin, methyl cellulose, carboxy methyl cellulose, or polyvinyl alcohol. Suspensions or solutions for intramuscular injection, together with the active compound, are pharmaceutically acceptable carriers such as sterile water, olive oil, ethyl oleate, glycols, for example, propylene glycol, and, if necessary, appropriate quantities of lidocaine hydrochloride.

Pharmaceutical compositions comprising cytotoxic T cells according to the present invention may be administered to a subject, for example, by venous injection (bolus injection) or continuous infusion. For example, the pharmaceutical composition according to the present invention may be administered at least 1 time, at least 2 times, at least 3 times, at least 4 times, or at least 5 times, continuously, or at specified time intervals, over at least 1 hour, at least 2 hours, at least 3 hours, at least 4 hours at least 8 hours, at least 12 hours, at least 1 day, at least 2 days, at least 3 days, at least 4 days, at least 5 days, at least 6 days, at least 7 days, at least 2 weeks, at least 3 weeks, at least 4 weeks, at least 1 month, at least 3 months, at least 6 months, or at intervals determined by clinical judgment. Injectable preparations may be formulated in ampoule form or in a unit dosage form with a multi-dose container. However, a person of ordinary skill in the art will understand that the dosage of the pharmaceutical composition according to the present invention may vary depending on various factors such as the subject's age, weight, height, sex, general medical condition and previous treatment history.

Pharmaceutical compositions according to the present invention may be formulated in sustained release form, and the sustained release formulation can be prepared by using a polymer that may be complexed with or may contain the cytotoxic T cells to be administered. For example, a matrix of poly (ethylene-co-vinyl acetate) or a matrix of stearic acid dimer and polyanhydride copolymer of sebacic acid may be used as a biosynthetic polymer.

As used in the present invention, the term "cancer" refers to any of the numerous diseases or disorders caused by abnormal, uncontrolled cell growth. The cells that may cause cancer are called cancer cells, and have unique typological characteristics such as uncontrolled proliferation, immortality, metastatic potential, rapid growth and proliferation. Often, cancer cells may be in the form of a tumor, but such cells may be present individually in mammals or may be non-tumor cells, such as leukemia cells. Cancer may be detected by a clinical or radiological method for detecting the presence of tumors; by testing cells from tumors or other biological samples obtained by means such as biopsies; by detecting cancer blood markers such as CA125, PAP, PSA, CEA, AFP, HCG, CA 19-9, CA 15-3, CA 27-29, LDH, and NSE; or by detecting cancer marker genotypes such as TP53 and ATM. However, a negative finding by an above method does not necessarily mean a non-cancer diagnosis: For example, a subject who has been found to have fully recovered from cancer may still have cancer; this is confirmed in the form of a relapse.

As used in the present specification, the term "about" may be understood within the range commonly used in the art, for example, within 2 standard deviations of the mean. "About" may be understood to mean within 50%, 45%, 40%, 35%, 30%, 25%, 20%, 15%, 10%, 9%, 8%, 7%, 6%, 5%, 4%, 3%, 2%, 1%, 0.5%, 0.1%, 0.05%, or 0.01% of the mentioned value.

The "composition" disclosed in this invention refers to a combination of the cytotoxic T cells according to the present invention as the active ingredient, and inactive ingredients such as natural or artificial carriers, labels or detectors, an active ingredients such as adjuvants, diluents, coupling agents, stabilizers, buffers, salts, lipophilic solvents, and preservatives, and comprises a pharmaceutically acceptable carrier. The carrier may also comprise pharmaceutical excipients and additional proteins, peptides, amino acids, lipids, and carbohydrates (for example, monosaccharides; disaccharides; trisaccharides; tetrasaccharides; oligosaccharides; alditol, aldonic acid, sugar-derived polysaccharides such as esterified sugar, or a sugar polymer or the like), alone or in combination, at 1 to 99.99 wt% or vol%. Protein excipients include, for example, human serum albumin, recombinant human albumin, gelatin, casein, and the like, but are not limited thereto. Representative amino acid components that may play a buffer role include, for example, alanine, arginine, glycine, betaine, histidine, glutamic acid, aspartic acid, cysteine, lysine, leucine, isoleucine, valine, methionine, phenylalanine, aspartame, and the like, but are not limited thereto. Carbohydrate excipients also include, for example, monosaccharides such as fructose, maltose, galactose, glucose, D-mannose, sorbose; disaccharides such as lactose, sucrose, trehalose, cellobiose; polysaccharides such as raffinose, maltodextrin, dextran, and starch; and alditols such as mannitol, xylitol, maltitol, lactitol, sorbitol, and myoinositol; but are not limited thereto.

A skilled person will be able to formulate the pharmaceutical composition of the present invention by methods known in the art. For example, as required, it may be used parenterally in the form of an injection of a sterile solution or suspension with water or another pharmaceutically acceptable liquid. For example, it may be appropriately combined with pharmaceutically acceptable carriers or media, in particular sterile water or saline solution, vegetable oil, emulsifier, suspension agent, surfactant, stabilizer, excipient, vehicle, preservative, binder and the like; it may be formulated by mixing in a unit-dosage form required by generally accepted pharmaceutical practice. The active ingredient amount used in the formulation is such that a suitable dosage in the indicated range may be obtained.

In addition, sterile compositions for injection may be formulated according to conventional formulation practice using excipient liquids, such as distilled water for injection. As the aqueous solution for injection may be used, for example, combinations of physiological saline; isotonic solutions containing glucose or other auxiliary agents, for example D-sorbitol, D-mannose, D-mannitol, sodium chloride, and suitable dissolution aids, for example alcohols, in particular ethanol, and polyalcohols, for example propylene glycol, polyethylene glycol; and nonionic surfactants such as polysorbate 80 (TM), HCO-50. Oily liquids include for example sesame oil and soybean oil, and may be used in combination with benzyl benzoate and benzyl alcohol as a dissolution aid.

Injection formulations may for example be administered by intravenous injection, intraarterial injection, selective intraarterial injection, intramuscular injection, intraperitoneal injection, subcutaneous injection, intraventricular injection, intracranial injection, intramedullary injection, and the like; preferably, however, they are administered by intravenous injection.

The composition of the present invention comprises a pharmaceutically effective amount of T cells. The effective amount may be readily determined by persons of ordinary skill in the art based on the disclosure in this specification. In general, a pharmaceutically effective amount is determined by 1st administering a low concentration of an active ingredient, and then gradually increasing the concentration until a desired effect is achieved in the subject without any side effects (for example, the symptoms associated with cancer are reduced or eliminated). Methods of determining appropriate dosages or intervals of administration for the administration of the compositions according to the present invention are described, for example, in Goodman and Gilman's The Pharmacological Basis of Therapeutics, Goodman et al., eds., 11th Edition, McGraw-Hill 2005, and Remington: The Science and Practice of Pharmacy, 20th and 21st Editions, Gennaro and University of the Sciences in Philadelphia, Eds., Lippencott Williams & Wilkins (2003 and 2005).

The method of administration of the composition according to the present invention may be determined based on various factors such as the subject's type of cancer, age, weight, sex, medical condition, severity of the disease, route of administration, and other medications to be administered separately. Accordingly, although the method of administration varies widely, it may be determined according to a commonly used method.

The amount of the composition according to the present invention to be administered to a subject may be determined by numerous factors such as the method of administration, subject's state of health, weight, and medical advice; all of these factors are within the scope of knowledge of a person of ordinary skill in the art.

The pharmaceutical composition for cancer prevention or treatment comprising the cancer antigen-specific cytotoxic T cell according to the present invention may comprise approximately 1 × 10⁶ cells/mL or more, approximately 2 × 10⁶ cells/mL or more, approximately 3 × 10⁶ cells/mL or more, approximately 4 × 10⁶ cells/mL or more, approximately 8 × 10⁶ cells/mL or more, approximately 6 × 10⁶ cells/mL or more, approximately 7 × 10⁶ cells/mL or more, approximately 8 × 10⁶ cells/mL or more, approximately 9 × 10⁶ cells/mL or more, approximately 1 × 10⁷ cells/mL or more, approximately 2 × 10⁷ cells/mL or more, approximately 3 × 10⁷ cells/mL or more, approximately 4 × 10⁷ cells/mL or more, approximately 5 × 10⁷ cells/mL or more, approximately 6 × 10⁷ cells/mL or more, approximately 7 × 10⁷ cells/mL or more, approximately 8 × 10⁷ cells/mL or more, approximately 9 × 10⁷ cells/mL or more, approximately 1 × 10⁸ cells/mL or more, approximately 2 × 10⁸ cells/mL or more, approximately 3 × 10⁸ cells/mL or more, approximately 4 × 10⁸ cells/mL or more, approximately 5 × 10⁸ cells/mL or more, approximately 6 × 10⁸ cells/mL or more, approximately 7 × 10⁸ cells/mL or more, approximately 8 × 10⁸ cells/mL or more, or approximately 9 × 10⁸ cells/mL or more, but a person of ordinary skill in the art may adjust the concentration of cytotoxic T cells within the range in which the same effects may be obtained.

It may also be combined with buffers, for example phosphate buffer solutions or sodium acetate buffer solutions; analgesics, for example procaine hydrochloride; stabilizers, for example benzyl alcohol, phenols and antioxidants. The prepared injection solution is usually charged into a suitable ampoule.

Suspensions and emulsions may contain as carriers, for example, natural gums, agar, sodium alginate, pectin, methyl cellulose, carboxy methyl cellulose, or polyvinyl alcohol. Suspensions or solutions for intramuscular injection, together with the active compound, are pharmaceutically acceptable carriers such as sterile water, olive oil, ethyl oleate, glycols, for example, propylene glycol, and, if necessary, appropriate quantities of lidocaine hydrochloride.

Pharmaceutical compositions comprising cytotoxic T cells according to the present invention may be administered to a subject, for example, by venous injection (bolus injection) or continuous infusion. For example, the pharmaceutical composition according to the present invention may be administered at least 1 time, at least 2 times, at least 3 times, at least 4 times, or at least 5 times, continuously, or at specified time intervals, over at least 1 hour, at least 2 hours, at least 3 hours, at least 4 hours at least 8 hours, at least 12 hours, at least 1 day, at least 2 days, at least 3 days, at least 4 days, at least 5 days, at least 6 days, at least 7 days, at least 2 weeks, at least 3 weeks, at least 4 weeks, at least 1 month, at least 3 months, at least 6 months, or at intervals determined by clinical judgment. Injectable preparations may be formulated in ampoule form or in a unit dosage form with a multi-dose container. However, a person of ordinary skill in the art will understand that the dosage of the pharmaceutical composition according to the present invention may vary depending on various factors such as the subject's age, weight, height, sex, general medical condition and previous treatment history.

Pharmaceutical compositions according to the invention may be formulated in sustained release form, and the sustained release formulation can be prepared by using a polymer that may be complexed with or may contain the cytotoxic T cells to be administered. For example, a matrix of poly (ethylene-co-vinyl acetate) or a matrix of stearic acid dimer and polyanhydride copolymer of sebacic acid may be used as a biosynthetic polymer.

As used in the present invention, the term "cancer" refers to any of the numerous diseases or disorders caused by abnormal, uncontrolled cell growth. The cells that may cause cancer are called cancer cells, and have unique typological characteristics such as uncontrolled proliferation, immortality, metastatic potential, rapid growth and proliferation. Often, cancer cells may be in the form of a tumor, but such cells may be present individually in mammals or may be non-tumor cells, such as leukemia cells. Cancer may be detected by a clinical or radiological method for detecting the presence of tumors; by testing cells from tumors or other biological samples obtained by means such as biopsies; by detecting cancer blood markers such as CA125, PAP, PSA, CEA, AFP, HCG, CA 19-9, CA 15-3, CA 27-29, LDH, and NSE; or by detecting cancer marker genotypes such as TP53 and ATM. However, a negative finding by an above method does not necessarily mean a non-cancer diagnosis: For example, a subject who has been found to have fully recovered from cancer may still have cancer; this is confirmed in the form of a relapse.

In the present invention, confirmation testing is preferably performed according to European Pharmacopeia 2.7.24 (flow cytometry), and confirmation is deemed to have occurred if at least 65% of CD8 + T cells are confirmed and at least 80% of CD8+ T cells are confirmed to have the CD45RO marker.

In the present invention, purity testing is preferably performed according to European Pharmacopeia 2.7.24 (flow cytometry), with the CD45RA percentage of CD8+ T cells set at 20% or less, the CD57 ratio at 35% or less, and PD-1 at 20% or less.

The cell function test or potency test in the present invention is preferably tested according to European Pharmacopoeia 2.7.24 (flow cytometry), with the IFN-Y expression rate in CD8 + T cells being at least 10%, or according to European Pharmacopoeia 2.7.24 (flow cytometry), with the LAMP1 expression rate in CD8 + T cells being 10% or more.

The cell viability test in the present invention is preferably performed according to the manual dye-exclusion method in European Pharmacopoeia 2.7.29 (nucleated cell count and viability), with the proportion of living cells being at least 70%.

In the present invention, the total cell count test is preferably performed according to the manual dye-exclusion method in European Pharmacopoeia 2.7.29 (nucleated cell count and viability), with the count of cells in 100 mL of finished product being 1.4 × 10⁹ cells/100 mL± 20% (1.12 - 1.68 × 10⁹ cells/100 mL).

In the present invention, the quality test may be preferably determined by a test prescribed in the standards and test methods for the raw drug and the finished drug of the cell therapeutic, but need not necessarily be determined by the relevant regulations or guidelines.

As used herein, the term "anti-cancer" encompasses "prevention" and "treatment"; "prevention" means any action in which cancer is inhibited or delayed by administration of a composition comprising the antibody of the present invention, and "treatment" means any action that improves or beneficially alters the symptoms of cancer by administering the antibody of the present invention.

Herein below, the present invention is explained in detail with reference to the following embodiments. These embodiments are intended to more concretely illustrate the present invention, and they do not limit the invention's scope.

### Practical Example 1: Pilot Production of EBV Specific CD8+ T Cell Therapeutics According to the Method of the Present Application (20-Day Process)

### 1-1. Proliferation of EBV Specific CD8+ T Cells

PBMCs were isolated from blood of an EBV-positive healthy individual as follows. 10 mL blood was slowly flowed into a 15 mL tube charged with 5 mL Ficoll (GE Healthcare, Ficoll paque plus, 17144002), and overlaid on top of the Ficoll solution. The tube was centrifuged at room temperature and 800xg for 20 minutes (braking speed: 0); only the white layer between the Ficoll and plasma was harvested, washed and used as PBMC. Autoplasma (autologous plasma) was isolated from the light yellow layer above PBMC, and filtered through a filter before use.

The isolated PBMCs were suspended at 1 × 10⁶ cells/mL in CTL medium (WELGENE, LM011-77) comprising 3% autologous plasma, and EBV peptide (Peptron) was added to reach a concentration of 2 µg/mL. . The cell suspension was aliquoted into 14 mL round tubes at 1 mL each, and incubation was begun in a CO₂ incubator (Nuaire, NU-5841).

On the second day of culture, 1 mL of CTL medium comprising 100 IU/mL IL-2 (Novartis, Proleukin) and 3% autologous plasma was added to each tube. On day 7 of culture, the 1 mL upper layer of medium was removed, and 1 mL of fresh CTL medium comprising 100 IU/mL IL-2 and 3% autologous plasma was added and incubated for another 2 days.

### 1-2. Selective Isolation of EBV-Specific CD8+ T Cells

On day 9 of culture, PBMCs in culture were harvested and washed in RPMI medium (WELGENE, LM011-01). The washed PBMCs were stained with anti-4-1BB antibody (Biolegend, 309818) and anti-CD8 antibody (Biolegend, 560347) at 4°C for 30 minutes, and then washed again with RPMI medium. PBMCs washed after staining were selectively isolated from cells expressing 4-1BB and CD8 using a flow cytometer (BD Biosciences, FACSMelody). Selectively isolated cells were washed with RPMI medium and counted with an automatic cell counter (Logos Biosystems, LUNA-FL). After suspending the isolated cells using ALyS505N medium (CELL SCIENCE & TECHNOLOGY INSTITUTE INC., 1020P10), the following steps were performed.

### 1-3. Mass Culture of Antigen-Specific CD8+ T Cells

Normal donor blood was irradiated to induce cell death, and PBMCs were isolated, suspended at 1 × 10⁷ cells/mL and frozen. Irradiated PBMCs (allogenic PBMCs) were used as a culture additive to provide the co-stimulation necessary to induce the proliferation of T cells. The isolated alloplasma was then separated from the light yellow layer on the upper layer of PBMC, filtered using a filter, and then used.

In a 50 mL tube, selectively isolated CD8+ T cells specific for EBV peptide and irradiated allogenic PBMCs, prepared at 200 times the isolated cell count, were suspended in 30 mL of ALys505N medium comprising 3% alloplasma. To this was added 1,000 IU/mL IL-2 and 30 ng/mL anti-CD3 antibody (BD Biosciences, 555336). 30 mL of the cell suspension was then placed in a 75T flask and incubation was begun in a CO₂ incubator.

On day 3 of culture, 20 mL of ALys505N medium comprising 1,000 IU/mL IL-2, 3% alloplasma was added to the 75T flask. On days 5 to 11 of culture, at 2-3 day intervals, the same quantity of ALys505N medium comprising 1,000 IU/mL IL-2, 3% alloplasma was added. On day 11 of culture, all cultured cells were harvested.

### Practical Example 2: Pilot Production of EBV-Specific CD8+ T Cell Therapeutics

### According to the Method of the Present Application (26-Day Process)

### 2-1. Proliferation of EBV Specific CD8+ T Cells

The 1-1 process of Practical Example 1 was carried out identically.

### 2-2. Selective Isolation of EBV-Specific CD8+ T Cells

On day 14 of culture, PBMCs in culture were harvested and washed in RPMI medium.

The washed PBMCs were counted with an automatic cell counter, and the cells were suspended in CTL medium to incubate at 2 × 10⁶ cells/mL each. 3% autoplasma and 100 IU/mL IL-2 were added to the CTL medium in which the cells were suspended, and then the suspended cells were placed in each well of a 24-well plate at 2 × 10⁶ cells per 1 mL. EBV peptides added on the first day of culture were added to reach a concentration of 2 ng/mL and were incubated in a CO₂ incubator.

On day 15 of culture, PBMCs in culture were harvested and washed in RPMI medium. The washed PBMCs were stained with anti-4-1BB antibody and anti-CD8 antibody at 4°C for 30 minutes, and then washed again with RPMI medium. PBMCs washed after staining were selectively isolated from cells expressing 4-1BB and CD8 using an automatic cell sorter. Selectively isolated cells were washed with RPMI medium and the cells were counted with an automatic cell counter. The isolated cells were suspended using ALyS505N medium and then the next step was performed.

### 2-3. Mass Culture of Antigen-Specific CD8+ T Cells

Normal donor blood was irradiated to induce cell death, PBMCs were isolated and suspended at 1 × 10⁷ cells/mL and then frozen and stored. Irradiated PBMC (allogenic PBMC) was used as a culture additive to provide the co-stimulation required to induce the proliferation of T cells. The isolated alloplasma was then separated from the light yellow layer on the upper layer of PBMC, filtered using a filter, and then used.

In a 50 mL tube, selectively isolated CD8+ T cells specific for EBV peptide and irradiated allogenic PBMCs, prepared at 200 times the isolated cell count, were suspended in 30 mL of ALys505N medium comprising 3% alloplasma. To this was added 1,000 IU/mL IL-2 and 30 ng/mL anti-CD3 antibody. 30 mL of the cell suspension was then placed in a 75T flask and incubation was begun in a CO₂ incubator.

On day 3 of culture, 20 mL of ALys505N medium comprising 1,000 IU/mL IL-2, 3% alloplasma was added to the 75T flask. On days 5 to 11 of culture, at 2-3 day intervals, the same quantity of ALys505N medium comprising 1,000 IU/mL IL-2, 3% alloplasma was added. On day 11 of culture, all cultured cells were harvested.

As described above, the confirmation and purity test results for the antigen-specific CD8+ T cells produced in the 26-day process confirmed that all the criteria shown in Table 1 were satisfied.

**[Table 1]**

| Test item | | Test criterion | Conventional production process (using plate coated with anti 4-1BB antibody) | New production process (using automatic cell sorter) |
|---|---|---|---|---|
| Total cell count test | | 7.0 × 10⁶ cell/mL + 10% 100 mL/infusion bag | 6.7x10⁶ cell/ml, 200ml, 2 bags | 6.9 × 10⁶ cell/ml, 200ml, 2 bags |
| Cell viability test | | >70% | ∼ 92.3% | ∼ 95.0% |
| Verification test | CD8T | >65% | 65.0% to 85.0% | ∼ 95.0% |
| | CD45RA | 20% of CD8T cells | ∼ 13.3% | ∼ 1.7% |
| | CD45RO | > 80% of CD8T cells | ∼ 98.5% | ∼ 99.7% |
| Purity test | CD57 | 35% of CD8T cells | ∼ 19.5% | ∼ 16.8% |
| | PD-1 | 20% of CD8T cells | ∼ 2.3% | ∼ 2.1% |
| | anti-CD3 mAb | Negative | Negative | Negative |
| Cell function test | IFN-Y production | > 10% of CD8T cells | ∼ 54.4% | ∼ 43% |
| | LAMP1 expression | > 10% of CD8T cells | -83.2% | -65% |
| Quality testing | Sterility test | Negative | - | - |
| | Endotoxin Test | < 1.0 EU/mL | - | - |
| | Mycoplasma test | Negative | - | - |
| | External virus suitability test | Negative | - | - |

In addition, the potency test result confirmed that the potency test criteria of Table 1 were satisfied, as shown in the table below.

### Practical Example 3: Freezing of semi-finished cells on day 15 of 20-day process

On day 15 of the whole process, half of the mass-cultured cells were collected, washed in RPMI medium and counted with an automatic cell counter. The cells were centrifuged at room temperature at 1,800 rpm for 5 minutes to remove supernatant and divided at 5 × 10⁵ cells/mL and the cells were then suspended in a CS-5 freezing medium (Biolife solution, 205102).

Cells that had been placed in a cell freezing tube at 5 × 10⁵ cell/mL were placed in a cell freezing container (Biocision, BCS-170) and kept in a -80°C deep freezer (ThermoFisher, IU28886D) for one day. The frozen cells were taken out again for one day and stored in a -196°C liquid nitrogen tank (Chart, 10650197) until the expiration date.

### Practical Example 4: Freezing of 20-day process finished product

At the end of incubation, all cultured cells were harvested, washed in RPMI medium and counted with an automatic cell counter.2 × 10⁷ cells were saved and used for phenotypic analysis and efficacy testing for testing the quality of the T cell therapeutics. The remaining cells were suspended in 5 mL of CS-5 freezing medium, counted with an automatic cell counter, and divided into 50 mL tubes for freezing at 3 × 10⁷ cells/mL and 5 × 10⁶ cells/mL cells. The cells in the in cell freezing tubes at 3 × 10⁷ cells/mL and 5 × 10⁶ cells/mL were placed in cell freezing containers and placed in -80°C cryogenic freezers for a period of one day. The cells that had been frozen for one day were taken out again and stored in a -196°C liquid nitrogen tank until the expiration date.

### Practical Example 5: Freezing of finished product of 26-day process

At the end of incubation, all cultured cells were harvested, washed in RPMI medium and counted with an automatic cell counter.2 × 10⁷ cells were saved and used for phenotypic analysis and efficacy testing for testing the quality of the T cell therapeutics. The remaining cells were suspended in 5 mL of CS-5 freezing medium, counted with an automatic cell counter, and divided into 50 mL tubes for freezing at 3 × 10⁷ cells/mL and 5 × 10⁶ cells/mL cells. The cells in the in cell freezing tubes at 3 × 10⁷ cells/mL and 5 × 10⁶ cells/mL were placed in cell freezing containers and placed in -80°C cryogenic freezers for a period of one day. The cells that had been frozen for one day were taken out again and stored in a -196°C liquid nitrogen tank until the expiration date.

### Practical Example 6: Thawing of frozen cells

### 6-1. Thawing after freezing of T cell therapeutic prepared by 20-day process

The cells stored in the -196°C liquid nitrogen tank were taken out, and the cells were thawed by soaking in a 37°C constant-temperature water bath (WB-2, Daehan Science Ltd., DH.WB000122) for a period from 2 minutes 30 seconds to 3 minutes. The thawed cells were transferred to a 15 mL tube, counted using an automatic cell counter, and the cell count and survival rate of the thawed cells were checked. 10 mL of warm RPMI medium was slowly overlaid on the thawed cells. The tube was centrifuged at room temperature at 1,800 rpm for 5 minutes to remove supernatant, and the cells were suspended in 1 mL of ALys505N medium. The number and survival rate of the cells suspended in 1 mL were again counted using an automatic cell counter, and the required number of cells were used.

The procedure of Practical Example 1 was carried out and after 2, 4, 8 and 12 weeks, the cells frozen by the procedure of Practical Example 4 were thawed as described above and the recovery rate and survival rate were measured; additionally, verification, purity, and potency tests were performed.

Upon thawing the 20-day finished product, as shown in Table 2 below, a recovery rate of at least 64% was confirmed, and the lowest survival rate was found to be approximately 77.0%.

**[Table 2]**

| Freezing period | Recovery rate (%) | Survival rate (%) |
|---|---|---|
| 2 weeks | 75+10 | 87 + 5 |
| 4 weeks | 72+10 | 83 + 5 |
| 8 weeks | 66+10 | 81 + 5 |
| 12 weeks | 64+10 | 77 + 5 |

In addition, the verification and purity test results confirmed that all respective standards were passed as shown in Table 3 below.

**[Table 3]**

| Freezing period | CD8 (%) (> 80%) | CD57: CD8 (%) (<35%) | PD-1: CD8 (%) (<20%) | CD45RA: CD8 (%) (<20%) | CD45RO: CD8 (%) (> 80%) |
|---|---|---|---|---|---|
| 0 weeks | 96.65 | 11.21 | 5.36 | 3.45 | 86.00 |
| 1: | 96.08 | 9.40 | 3.80 | 0.74 | 92.97 |
| 2 weeks | 95.40 | 10.95 | 4.88 | 0.93 | 88.18 |
| 3 weeks | 90.60 | 12.49 | 6.65 | 0.93 | 86.66 |
| 4 weeks | 98.91 | 10.04 | 2.49 | 0.73 | 93.95 |
| 8 weeks | 99.5 | 11.02 | 3.45 | 1.22 | 94.83 |
| 12 weeks | 98.83 | 12.61 | 11.23 | 1.21 | 96.65 |

In addition to the above experiments, it was also confirmed that the results of the potency test passed the respective standards (Table 4).

**[Table 4]**

| Freezing period | LAMP-1 (%) (> 10%) | IFN-g (%) (> 10%) |
|---|---|---|
| 0 weeks | 15.15 | 16.33 |
| 1: | 12.34 | 15.08 |
| 2 weeks | 23.22 | 23.24 |
| 3 weeks | 18.07 | 20.42 |
| 4 weeks | 22.26 | 34.75 |
| 8 weeks | 28.62 | 15.75 |
| 12 weeks | 19.28 | 14.86 |

### 6-2. Thawing after freezing of T cell therapeutic prepared by 26-day process

The procedure of Practical Example 2 was carried out and after 2 and 4 weeks, the cells frozen by the procedure of Practical Example 5 were subjected to the procedure of Practical Example 6 to measure the recovery rate and survival rate; additionally, verification, purity, and potency tests were performed.

Upon thawing the 26-day finished product, as shown in Table 5 below, an 80% recovery rate was confirmed, and the survival rate was at least 91%.

**[Table 5]**

| Freezing period | Recovery rate (%) | Survival rate (%) |
|---|---|---|
| 2 weeks | 80+10 | 91 + 10 |
| 4 weeks | 80+10 | 91 + 10 |

In addition, the verification and purity test results confirmed that the internal standards were passed as shown in Table 6 below.

**[Table 6]**

| Freezing period | CD8 (%) (> 80%) | CD57: CD8 (%) (<35%) | PD-1: CD8 (%) (<20%) | CD45RA: CD8 (%) (< 20%) | CD45RO: CD8 (%) (> 80%) |
|---|---|---|---|---|---|
| 0 weeks | 97.47 | 4.60 | 6.97 | 1.03 | 98.49 |
| 2 weeks | 98.4 | 5.9 | 15.3 | 0.6 | 92.2 |
| 4 weeks | 98.5 | 7.4 | 8.4 | 0.8 | 98.5 |

It was also confirmed that the potency test results passed the respective standards as shown in Table 7 below.

**[Table 7]**

| Freezing period | LAMP-1 (%) (> 10%) | IFN-g (%) (> 10%) |
|---|---|---|
| 0 weeks | 22.28 | 24.19 |
| 2 weeks | 15.7 | 21 |
| 4 weeks | 15.3 | 24 |

### Practical Example 7: Mass Culturing Process Using Frozen Cells

Normal donor blood was irradiated to induce cell death, PBMCs were isolated and suspended at 1 × 10⁷ cells/mL and then frozen and stored. Irradiated PBMC (allogenic PBMC) was used as a culture additive to provide the co-stimulation required to induce the proliferation of T cells.

Cells were thawed according to the frozen cell thawing method of Practical Example 6 above, counted, and suspended in 30 mL of ALys505N medium comprising 3% alloplasma. To this, cells irradiated with allogenic PBMC were added in a quantity 200 times the number of thawed cells, and 1,000 IU/mL IL-2 and 30 ng/mL anti-CD3 antibody were added. All the cells to which additives had been added were transferred to a 75T flask and incubated in a CO2 incubator. On day 3 of culture, 20 mL of ALys505N medium comprising 1,000 IU/mL IL-2 and 3% alloplasma was added to the 75T flask. On day 5 of culture, 50 mL of ALys505N medium comprising 1,000 IU/mL IL-2, 3% alloplasma was added to the 75T flask. On day 7 of culture, 100 mL of ALys505N medium comprising 1,000 U/mL IL-2, 3% alloplasma was prepared and transferred to a 175T flask together with the cultured cells. On day 9 of culture, 200 mL of ALys505N medium comprising 1,000 U/mL IL-2, 3% alloplasma was added, and cultured in two 175T flasks. On day 11 of culture, all cultured cells were harvested.

Upon performing the procedure of Practical Example 7, one, two and three months after performing the procedure of Practical Example 3, the cell survival rate was confirmed to be at least 92% after mass culture as shown in Table 8 below, and it was found that the cell proliferation had reached a factor of at least 4800.

**[Table 8]**

| Freezing period | Survival rate of mass cultured cells (%)> 70% | 4000-fold total factor of increase for mass-cultured cells |
|---|---|---|
| 1 month | 99 | 12000 |
| 2 months | 93 | 7000 |
| 3 months | 92 | 4800 |

In addition, the verification and purity tests showed that all the respective standards were passed, as shown in Table 9 below.

**[Table 9]**

| Freezing period | CD8 (%) (>80%) | CD57: CD8 (%) (<35%) | PD-1: CD8 (%) (<20%) | CD45RA: CD8 (%) (<20%) | CD45RO: CD8 (%) (> 80%) |
|---|---|---|---|---|---|
| 1 month | 95.72 | 16.94 | 1.77 | 1.75 | 99.05 |
| 2 months | 90.81 | 8.32 | 1.10 | 0.63 | 98.24 |
| 3 months | 95.57 | 15.30 | 0.45 | 2.91 | 98.40 |

In addition, the results of the potency test also all passed the respective standards as shown in Table 10 below.

**[Table 10]**

| Freezing period | LAMP-1: CD8 (%) (> 10%) | IFN-g: CD8 (%) (> 10%) |
|---|---|---|
| 1 month | 40.81 | 21.94 |
| 2 months | 19.77 | 15.68 |
| 3 months | 14.18 | 17.70 |

After performing the procedure of Practical Example 4, upon performing the procedure of Practical Example 7 after 4, 8, and 12 weeks, the cell survival rate was confirmed to be 89% or more after mass culture as shown in the following table, and it was found that the cell proliferation had reached a factor of 175 or more.

**[Table 11]**

| Freezing period | Survival rate (%) | Cell proliferation factor |
|---|---|---|
| 4 weeks | 89.1 | 175.4 |
| 8 weeks | 96.27 | 1291 |
| 12 weeks | 93.52 | 988 |

In addition, the verification and purity tests showed that all the respective standards were passed, as shown in Table 12 below.

**[Table 12]**

| Freezing period | CD8 (%) (>80%) | CD57: CD8 (%) (<35%) | PD-1: CD8 (%) (<20%) | CD45RA: CD8 (%) (< 20%) | CD45RO: CD8 (%) (> 80%) |
|---|---|---|---|---|---|
| 4 weeks | 94.92 | 2.57 | 1.21 | 2.13 | 94.67 |
| 8 weeks | 98.76 | 1.90 | 1.32 | 0.47 | 99.57 |
| 12 weeks | 95.29 | 2.67 | 4.83 | 4.10 | 99.55 |

In addition, the results of the potency test also passed their respective standards as shown in Table 13 below.

**[Table 13]**

| Freezing period | LAMP-1 (%) (> 10%) | IFN-g (%) (> 10%) |
|---|---|---|
| 4 weeks | 22.11 | 41.28 |
| 8 weeks | 20.43 | 21.51 |
| 12 weeks | 26.29 | 30.00 |

For example, for claim construction purposes, it is not intended that the claims set forth below be construed in any way narrower than the literal language thereof, and it is thus not intended that exemplary embodiments from the specification be read into the claims. Accordingly, it is to be understood that the present invention has been described by way of illustration and not by way of limiting the scope of the claims. Accordingly, the present invention is limited only by the following claims. All publications, issued patents, patent applications, books and journal articles, cited in this application are each incorporated into the present application by reference in their entirety.

## Claims

1. Method of preparing cancer antigen-specific CD8+ T cells, comprising
(a) activating the CD8+ T cells from among PBMCs comprising cancer antigen-specific CD8+ T cells, and then expressing a co-stimulatory factor of these CD8+ T cells;
(b) selecting the CD8+ T cells expressing a co-stimulatory factor among the cells activated in step (a);
(c) increasing the number of CD8+ T cells selected in step (b); and
(d) freezing the CD8+ T cells obtained in step (c).

2. Method of preparing cancer antigen-specific CD8+ T cells according to Claim 1, further comprising a step (e) of thawing the CD8+ T cells that were frozen in step (d).

3. Method of preparing cancer antigen-specific CD8+ T cells according to Claim 1, wherein the cancer antigen is at least one selected from the group made up of hTERT, NY-ESO1, MAGE-A3, WT1 and EBV.

4. Method of preparing cancer antigen-specific CD8+ T cells according to Claim 1, wherein step (b) is performed using a closed-system flow cytometer.

5. Method of preparing cancer antigen-specific CD8+ T cells according to Claim 1, wherein in step (d), freezing is performed when the number of CD8+ T cells expressing a co-stimulatory factor is greater than or equal to a predetermined number of cells per mL.

6. Method of preparing cancer antigen-specific CD8+ T cells according to Claim 1, wherein in step (d), freezing is perfumed when the number of CD8+ T cells is at least a predetermined number of cells per mL, and the proportion of CD8+ T cells expressing the co-stimulatory factor is at least a predetermined ratio.

7. Method of preparing cancer antigen-specific CD8+ T cells according to Claim 1, wherein step (d) comprises a step of detecting the number of CD8+ T cells and the proportion of CD8+ T cells expressing a co-stimulatory factor.

8. Method of preparing cancer antigen-specific CD8+ T cells according to Claim 2, further comprising a step (f) of increasing the number of CD8+ T cells obtained in step (e).

9. Method of preparing cancer antigen-specific CD8+ T cells according to Claim 1, wherein the co-stimulatory factor is one or more selected from the group made up of CD28, CTLA-4, ICOS, PD1, BTLA, HVEM, CD27, OX40, 4-1BB, CD30, and SLAM.

10. Method of preparing a cancer treatment composition, comprising
(a) activating the CD8+ T cells from among PBMCs comprising cancer antigen-specific CD8+ T cells, and then expressing a co-stimulatory factor of these CD8+ T cells;
(b) selecting the CD8+ T cells expressing a co-stimulatory factor among the cells activated in step (a);
(c) increasing the number of CD8+ T cells selected in step (b); and
(d) freezing the CD8+ T cells obtained in step (c).

11. Method of preparing a cancer treatment composition according to Claim 10, further comprising a step (e), thawing the CD8+ T cells that were frozen in step (d).

12. Cancer treatment composition comprising cancer antigen-specific CD8+ T cells prepared by steps comprising
(a) activating the CD8+ T cells from among PBMCs comprising cancer antigen-specific CD8+ T cells, and then expressing a co-stimulatory factor of these CD8+ T cells;
(b) selecting the CD8+ T cells expressing a co-stimulatory factor among the cells activated in step (a);
(c) increasing the number of CD8+ T cells selected in step (b); and
(d) freezing the CD8+ T cells obtained in step (c).

13. Cancer treatment composition according to Claim 12, further comprising a step (e), thawing the CD8+ T cells that were frozen in step (d).

14. Cancer treatment method comprising a step of administering a pharmaceutically effective amount of a cancer treatment composition comprising cancer antigen-specific CD8+ T cells prepared by steps comprising
(a) activating the CD8+ T cells from among PBMCs comprising cancer antigen-specific CD8+ T cells, and then expressing a co-stimulatory factor of these CD8+ T cells;
(b) selecting the CD8+ T cells expressing a co-stimulatory factor among the cells activated in step (a);
(c) increasing the number of CD8+ T cells selected in step (b); and
(d) freezing the CD8+ T cells obtained in step (c).

15. Cancer treatment method according to Claim 14, further comprising a step (e), thawing the CD8+ T cells that were frozen in step (d).
